(19) Europäisches Patentamt
European Patent Office
Office européen des brevets

(11) **EP 4 223 883 A1**

(12) **EUROPEAN PATENT APPLICATION**

(43) Date of publication:
**09.08.2023 Bulletin 2023/32**

(21) Application number: **22154810.0**

(22) Date of filing: **02.02.2022**

(51) International Patent Classification (IPC):
***C12P 17/10*** *(2006.01)* ***A61K 31/675*** *(2006.01)*
***A61P 25/00*** *(2006.01)* ***C07D 209/16*** *(2006.01)*
***C12N 9/02*** *(2006.01)* ***C12N 9/10*** *(2006.01)*
***C12N 9/12*** *(2006.01)* ***C12N 9/88*** *(2006.01)*

(52) Cooperative Patent Classification (CPC):
**C12P 17/10; A61P 25/00; C07D 209/16; C07F 9/5728; C12N 9/0071; C12N 9/0083; C12N 9/1007; C12N 9/1085; C12N 9/1205; C12N 9/88; C12Y 201/01049; C12Y 205/01006; C12Y 207/01; C12Y 401/01028**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Infinit Biosystems**
**10115 Berlin (DE)**

(72) Inventors:
- **JARA CRUA, Federico**
  **10557 Berlin (DE)**
- **ROLLI PACHECO, Rafael**
  **10557 Berlin (DE)**
- **METZ, Carlo**
  **10557 Berlin (DE)**

(74) Representative: **KATZAROV S.A.**
**Geneva Business Center**
**12 Avenue des Morgines**
**1213 Petit-Lancy (CH)**

# (54) PROCESS FOR PRODUCING TRYPTAMINES

(57) The present invention relates to a cell-free process to enzymatically produce tryptamines from L-tryptophan and to a reactor or a kit of reactors for performing such process.

[Fig. 5]

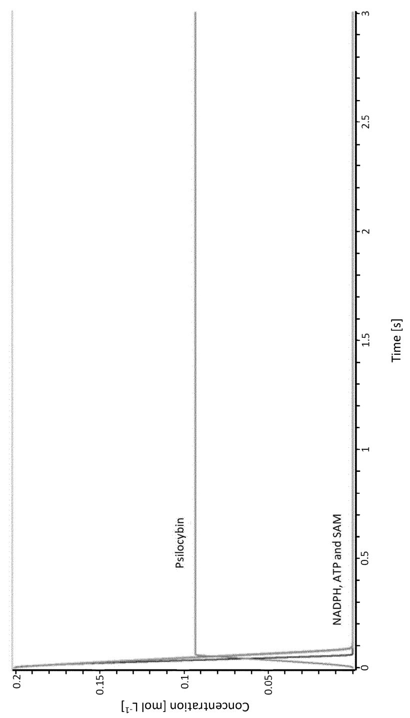


EP 4 223 883 A1

## Description

### Technical Field

The present invention relates to a cell-free process to enzymatically produce tryptamines from L-tryptophan and to a reactor or a kit of reactors for performing such process.

### Background Art

Mental health is becoming one of the major problems of modern society and concerns are on the rise as many aren't finding relief from their symptoms and much less a cure for the causes. Currently we rely on pharmacological drugs to numb or repress symptoms, such as selective serotonin reuptake inhibitors (SSRIs). Such treatments however are associated with unwanted side effects and there is therefore a need to seek for alternative solutions to improve adverse mental conditions, such as persistent or severe depression.

Naturally occurring psychedelics, such as tryptamines, have been used for thousands of years by humans, and possibly other species of humanoids, for recreational, healing and spiritual purposes. These substances have the capacity to induce intense shifts in consciousness and cognition through transient changes in emotions, perceptual processing, one's sense of self, and feelings of connectedness (Aday et al. 2020). Scientific research into their effects grew through the early part of the 20th century before their prohibition, which made them illegal in most of the world after the 1970s, and caused drastic decrease of the research activities in this field. A break-through happened in the early 1990s, as serotonergic psychedelics, such as tryptamines, were rediscovered to treat different psychiatric disorders. Research on these compounds has been re-established by different groups, culminating in new centers for psychedelic research at prestigious academic institutions like Imperial College London and Johns Hopkins University, and every year medical centers around the globe are showing the power of psychedelics together with integrated psychotherapies to completely revert symptoms of many diseases (Nutt et al. 2020). This opened a new era for mental health treatments, characterized by shifting pharmacological medication with substances and therapies that restore balance to the brain.

Tryptamines are a group of monoamine alkaloids (indolalkylamines), derived from the amino acid tryptophan. The basic element of tryptamines is the indole structure.

Tryptamines can be found in a wide range of natural sources including plants, fungi, microbes, and amphibia. Tryptamine itself and N,N-dimethyltryptamine are endogenous in the human system. Serotonin and melatonin are two essential tryptamines present as neurotransmitters in the brain.

Among tryptamine derivatives which are of growing interest, one can cite psilocybin, which is a psychedelic compound found naturally in varying concentrations in over 200 species of *Basidiomycota* mushrooms. Psilocybin is advantageously used as a prodrug capable of releasing the active compound psilocin upon ingestion. Psilocin has been identified as a partial agonist of the human 5-$HT_{2A}$-receptor and medical indications such as treatment of depressive disorders, anxiety, cancer-related existential distress and substance dependencies are envisaged.

The structures of tryptamine and of the triptamines N,N-dimethyltryptamine, psilocybin and psilocin are provided in [Table 1] below:

[Table 1]

| Compound name | Structure |
|---|---|
| Tryptamine | 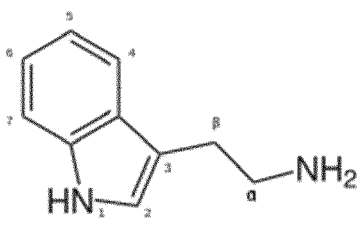 |

(continued)

| Compound name | Structure |
|---|---|
| Psilocybin | 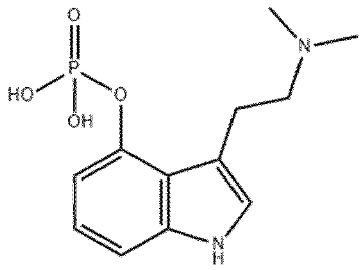 |
| Psilocin | 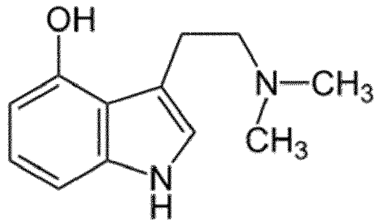 |
| N,N-dimethyltryptamine (DMT) | 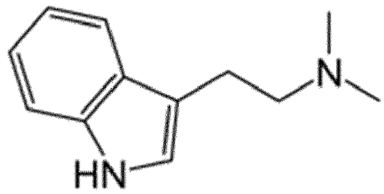 |

As the promising results from clinical trials continue to expand, demand for tryptamines rises, pushing for the development of more efficient manufacturing methods. Tryptamines are currently obtained by chemical synthesis, isolated from natural sources, or produced in recombinant hosts organism.

Purification from natural animal, vegetable, or fungal sources involves considerable efforts, from harvesting to extraction, to purification and isolation. Such processes are long, expensive, and technically complex. In particular, psilocybin is present at low percentages in most mushroom varieties, which makes it particularly expensive to obtain this compound from natural sources and the yields are low. Furthermore, psychedelics obtained by purification from natural sources are not consistent and fail to comply with medical regulations. It is in particular very difficult to obtain tryptamines from natural sources with sufficient purity.

The growing interest in tryptamines caused research to dig into the innate mechanisms involved in the production of such compounds in their natural source organisms and the main enzymes responsible for the formation of these compounds have been identified.

For example, the enzymes involved in the production of psilocybin in *Psilocybe* mushrooms have been identified (Fricke et al, 2017). The enzyme PsiD catalyzes the decarboxylation of L-tryptophan to produce tryptamine. The carbon in position 4 of the benzene ring of tryptamine is then substituted with an alcohol by the enzyme PsiH to form 4-hydroxytryptamine. The enzyme PsiK then phosphorylates 4-hydroxytryptamine to form norbaeosystin. The primary amine is also methylated twice by the enzyme PsiM. Psilocybin can then be converted to psilocin by reverse action of the enzyme PsiK. Following the discovery of the enzymes involved in psilocybin biosynthesis, later studies investigated enzymatic production of psilocybin. Fricke et al. 2017 first disclosed the *in-vitro* biosynthetic production of psilocybin from L-tryptophan using the recombinant enzymes PsiD, PsiH, PsiK and PsiM.

Fricke et al 2020 describes a process for the production of psilocybin using a process combining chemical synthesis and an enzymatic *in-vitro* phosphorylation step using the enzyme PsiK. Although this study concludes that such a process may provide access to psilocybin at a potentially reasonable cost, there is still a need to provide for an even more efficient process that is easier to scale-up.

The role of cofactors in the biosynthesis of psilocybin was investigated in Demmler et al. 2020. This study describes that synthesis of psilocybin from L-tryptophan involves one equivalent of 5'-adenosine triphosphate (ATP) and two equivalents of S-adenosyl-L-methionine (SAM). It also suggests that nucleoside cofactor salvage would be required to maintain high psilocybin production rates in vitro. This study further demonstrated that removal of SAH, which is released after methylation of the amine using SAM as a cofactor, increased the activity of PsiM and therefore allowed

for more efficient production of psilocybin. For SAH removal, the enzyme SAHH and AdoK, which are naturally present in *Psilocybe* mushroom, were used.

Soon after the identification of the enzymes involved in psilocybin biosynthesis, processes have also been developed for producing tryptamines by heterologous production in recombinant host cells. For example, WO2019/180309A1, WO2019/173797, WO2021/086513A1 and US2021147888A1 disclose methods for producing psilocybin by growing recombinant host cells comprising polynucleotides encoding PsiD, PsiH, PsiK and PsiM.

Unlike other psychedelics, DMT is endogenously produced by the central nervous system of animals including humans. In addition, this compound is found in a variety of plants such as in the genera *Phalaris, Delosperma, Acacia, Desmodium, Mimosa, Virola,* and *Psychotria.* The enzyme indolethylamine-N-methyltransferase (INMT) has been identified in Dean, 2018 as being able to catalyze the dimethylation of the amine of L-tryptophan such as to form N,N-dimethyltryptamine (DMT). DMT can be derivatized by adding a radical on the carbon atom in position 5 of the benzene ring of DMT, such as to form 5-hydroxy-DMT, 5-methoxy-DMT, 5-ethoxy-DMT and 5-bromo-DMT. DMT and its derivatives mentioned above interact with the 5-HT and other receptors in the brain to produce their psychedelic and therapeutic effect.

DMT has not been produced in recombinant hosts so far. This makes the need for the development of more efficient production methods even more acute.

Manufacturing is starting to shift to more advanced and efficient ways to obtain organic psychedelics through biosynthesis. There is therefore a need to further enhance biosynthetic processes for the production of psilocybin, psilocin and DMT. In particular, it is sought to provide integrated methods that allow for substantially complete and high-efficiency conversion of inexpensive substrates into the desired tryptamine derivative of interest. It is further desired to provide economically competitive methods characterized by high yields and which can run autonomously for the whole duration of the reaction. It is further sought to produce tryptamines with a degree of purity and a level of safety that makes it possible to use such products for therapeutic purpose as pharmaceutical compositions.

To make bio-derived products economically competitive high-efficiency conversion of input biomass into the desired compound is required. For the production of organic biosynthesized molecules, we believe that the way to go is to get inspired and mimic the biotransformations, environment, and rhythm of cellular pathways, rather than trying to force and control life itself. The aim of the present process is to adapt biology to the purpose of industrial production and develop a simpler, more streamlined version for successful bioproduction. The present invention aims at solving these problems and proposes a cell-free metabolic engineering tryptamine system for the biosynthetic production of both psilocybin, psilocin, and DMT.

## Summary of Invention

In a first aspect, the invention relates to a method for producing a tryptamine derivative of Formula I

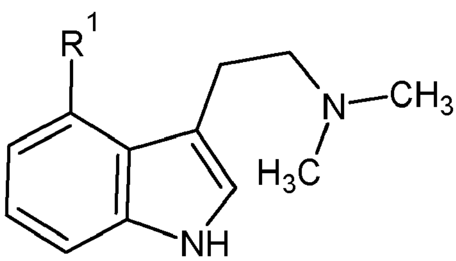

I

wherein R1 is selected from -H, $-OPO(OH)_2$, and -OH;

and wherein the tryptamine derivative of Formula I is selected from the group consisting of psilocybin, psilocin and N,N-dimethyltryptamine (DMT);

by a cell-free biosynthesis process using L-tryptophan as a substrate, comprising

a) enzymatically converting L-tryptophan to tryptamine having the Formula II, wherein $R^1$ is hydrogen

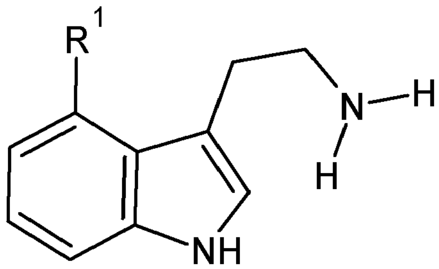

II

using at least one enzyme having an amino acid decarboxylase activity;

b) optionally enzymatically substituting the hydrogen atom in position $R^1$ of tryptamine of Formula II with a radical selected from $-OPO(OH)_2$, and -OH using at least one suitable enzyme and at least one suitable donor,

c) enzymatically dimethylating the primary amine of a compound of Formula II as obtained at the end of step a) or step b), such as to produce a compound of Formula I, by using at least one methyltransferase and using two molecules of S-adenosyl methionine (SAM) as methyl donors, thus producing S-adenosyl cysteine (SAH) as a by-product;

wherein the method further comprises continuously recycling the methyl donor SAM and the cofactor ATP by

1) enzymatically cleaving the SAH produced in step c) into adenosine and L-homocysteine using a hydrolase, preferably the enzyme SAHH;

2) enzymatically phosphorylating adenosine using a kinase, preferably the enzyme AdoK, and adenosine triphosphate (ATP) as a phosphate donor, thus producing adenosine monophosphate (AMP) and adenosine diphosphate (ADP);

3) enzymatically phosphorylating the ADP and the AMP obtained in step 2) using a kinase and polyphosphate as a phosphate donor, such as to form ADP from AMP and ATP from ADP;

4) enzymatically phosphorylating the ADP produced in step 3) using a kinase and polyphosphate as a phosphate donor such at to form ATP;

5) enzymatically reacting L-methionine and ATP using a transferase, preferably a S-adenosyl methionine synthetase, preferably the enzyme SAMS, such as to generate SAM and tripolyphosphate;

wherein polyphosphate is provided during the process in an amount sufficient to provide at least n available phosphate groups per molecule of L-tryptophan used as starting material,

wherein n= 6 + [number of phosphate groups present in $R^1$ of the compound of Formula I]; and

wherein L-methionine is provided in an amount such that the L-methionine to L-tryptophan ratio is of at least 2:1.

In a second aspect, the invention relates to a reactor or a kit of reactors for producing a compound of Formula I

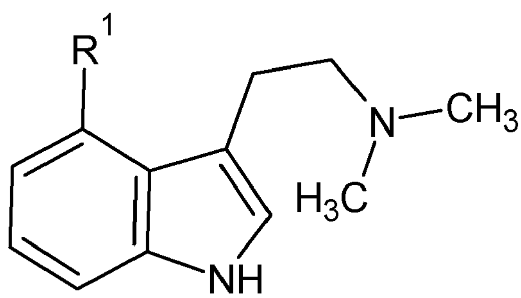

I

wherein $R^1$ is selected from -H, $-OPO(OH)_2$, and -OH;
and wherein the tryptamine derivative of Formula I is selected from the group consisting of psilocybin, psilocin and N, N-dimethyltryptamine (DMT); comprising

1) a reactor I comprising

a) L-tryptophan;

b) at least one amino acid decarboxylase;

2) optionally a reactor II comprising

a) at least one enzyme suitable for substituting a hydrogen atom in position R1, of a compound of Formula II with another radical selected from -OHPO3- and -OH;

b) at least one donor suitable for forming a radical selected from -OHPO3- or -OH; and

c) a recycling valve suitable to recycle the at least one donor;

3) a reactor III comprising

a) at least one methyltransferase

b) S-adenosyl methionine (SAM) as methyl donor,

c) A SAM recycling valve comprising

i) at least one hydrolase, preferably a S-adenosyl homocysteine hydrolase,

ii) at least one kinase, preferably a polyphosphate kinase and an adenosine kinase,

iii) L-methionine

iv) ATP

v) a transferase capable of transferring an adenosyl group from ATP to L-methionine, preferably an S-adenosylmethionine synthase (SAMS); and

vi) polyphosphate,

wherein any combination of two or more of reactors I, II, and III can be merged in one single reactor.

**Brief Description of Drawings**

[Fig.1] is a schematic representation of the method of the present invention for the production of psilocybin.

[Fig. 2] is a schematic representation of the method of the present invention for the production of N,N-dimethyltryptamine.

[Fig. 3] is a graphical representation of the Michaelis-Menten saturation curve for an enzyme reaction showing the relation between the substrate concentration ([S]) and the reaction rate. Increasing the concentration of the substrate, the rate of the process v increases, until a maximum value (Vmax): physically when this limit is achieved all the enzymes present in solution are saturated with the substrate, and increasing [S] has no longer effects on the global rate. The system achieves the half-maximum velocity ½ Vmax when [S] = Km.

[Fig. 4] is a graph showing the time course evolution simulation of all substrates and cofactors in the enzymatic

synthesis of psilocybin.

[Fig. 5] is a zoom on the lower part of the graph of [Fig. 4], wherein the curves showing the evolution of the concentration of L-tryptophan and of tryptamine have been removed, such as to show more clearly the evolution of the concentration of psilocybin.

**Detailed description**

The process of the present invention is the first complete and scalable biosynthetic enzymatic cell-free process providing for the complete pathway from L-tryptophan to psychedelic tryptamines. Even though the enzymes catalyzing the formation of such tryptamines have been identified in the prior art, the present invention for the first time reduces the process to practice, in such a way that is suitable for industrial production. This process is made particularly advantageous by the setting up of a complete and optimized recycling valve for regenerating continuously and autonomously the cofactors SAM and ATP, such that the system can run constantly and automatically to transform all the substrate into the desired tryptamine derivative.

The process is performed in at least one biosynthesis reactor and does not involve any living cell or organism. The process of the present invention is advantageous in that it starts from the simple, easy to source and inexpensive substrate L-tryptophan. L-tryptophan is converted into a tryptamine derivative of interest, using exclusively enzymatic reactions. Such reactions are catalyzed by defined enzymes. Each of such enzymes shall be interpreted as referring also to natural or artificial variants of such enzymes and homologous enzymes, provided that the described function of the enzyme is retained. Such variants shall include those generated by natural evolution or obtained by genetic engineering.

The overall pathway leading from L-tryptophan to desired tryptamine derivatives of Formula I involves at least two enzymatic reactions: the decarboxylation of L-tryptophan and the double methylation (dimethylation) of the primary amine to form a tertiary amine. When only these two reactions are performed, the tryptamine derivative obtained is N, N-dimethyltryptamine (DMT). It is possible to obtain other tryptamines, namely psilocybin and psilocin by substituting the hydrogen atom on the benzene ring in position $R^1$ with another radical by performing additional optional reactions, as described below.

The first step is always the decarboxylation of L-tryptophan to form tryptamine. Such reaction is catalyzed by an amino acid decarboxylase, preferably an L-amino acid decarboxylase, more preferably an aromatic L-amino acid decarboxylase, preferably a tryptophan decarboxylase. Said amino acid decarboxylase is preferably selected from AADC, PsiD, TDC and mixtures thereof. AADC (EC:4.1.1.28) is an aromatic L-amino acid decarboxylase that can be obtained from a wide range of organisms, including animals and plants. PsiD (EC:4.1.1.105) is an L-tryptophan decarboxylase that can be obtained from fungi of the genus *Psilocybe.* TDC (EC:4.1.1.28) is a tryptophan decarboxylase found in a variety of plants. Any of AADC, PsiD, TDC, or combinations thereof can be used to decarboxylate L-tryptophan, irrespective of the final product that is to be produced.

The double methylation of the primary amine to form a tertiary amine is catalyzed by a methyltransferase. When the process is for producing psilocin or psilocybin the methyltransferase is preferably PsiM (EC:2.1.1.345). When the process is for producing DMT, the methyltransferase is preferably INMT (EC:2.1.1.49). Two units of the cofactor S-Adenosyl methionine (SAM) are required for this step.

The substitution of the hydrogen in $R^1$ by an -OH group is catalyzed by a monooxygenase, preferably a cytochrome P450-containing monooxygenase. The - OH group can then be phosphorylated by a kinase.

After tryptamine is formed, this compound can be directly subjected to dimethylation of the amine to form DMT. Alternatively, the hydrogen in position $R^1$ of the benzene ring can be substituted by a phosphate before the amine is dimethylated.

The substitution in position $R^1$ of the benzene ring can be performed before or after methylation of the amine. It is however preferred to perform the substitution before the dimethylation of the amine. This improves the selectivity of the methylation reaction and thus the efficiency of the process. Therefore, in a preferred embodiment, psilocybin is formed by substituting the hydrogen in position $R^1$ of tryptamine with a phosphate group before the methylation of the amine.

Therefore, in a preferred embodiment, the hydrogen in $R^1$ of tryptamine is substituted with an -OH group to form 4-hydroxytryptamine by a monooxygenase, preferably a cytochrome P450-containing monooxygenase, more preferably PsiH (EC: 1.14.99.59). PsiH carries a dependency functionality on a cytochrome P450 reductase (Cpr) which aids PsiH with electron transfer from NADPH. The -OH group is then be phosphorylated by a kinase, preferably the 4-hydroxytryptamine kinase PsiK, (EC:2.7.1.222) to form norbaeocystin. After the substitution on the benzene ring, the amine is dimethylated by a methyltransferase, preferably PsiM. Figure 1 provides the complete process pathway for this preferred process for the production of psilocybin.

For the production of psilocin, psilocybin shall first be produced and psilocybin is then dephosphorylated, either

spontaneously, for example in neutral or acidic conditions, or enzymatically, for example by a phosphorylase or by PsiK, using one ATP molecule.

The reactions of decarboxylation, methylation of the amine, and substitution on the benzene ring may be performed in a one-pot process, wherein the substrate and the enzymes required for all reactions are provided at the beginning of the reaction.

Alternatively, the reactions may be performed in a one-pot reactor, with part of the enzymes and substrates being added after a certain reaction time, such as to allow for part of the substrate to react before the addition of the enzymes involved in a later stage of the process. In another alternative, different reactions are performed in different reactors, such as to isolate one or more reaction(s) from other reaction(s), such as to avoid interference between reactions. The addition of enzymes at a second point in time or the use of separate reactors are more burdensome than a one-pot reaction but such solutions positively impact the reaction yield by isolating a reaction from competitive reactions.

In an embodiment, when performing the substitution on the benzene ring before methylating the amine the enzyme(s) required to perform the substitution on the benzene ring are added to the reactor before adding the enzyme(s) necessary to methylate the amine.

The enzymes can be provided in the form of isolated enzymes or as part of a cell-free matrix. Preferably, the enzymes are provided as isolated enzymes. In the alternative where an enzyme is provided as part of a matrix, different types of matrices are suitable for the purpose of the present invention. One suitable type of cell-free matrix in which the necessary enzymes can be provided is a cell lysate or a fraction of a cell lysate.

Cell lysates suitable as sources of enzymes for the purpose of the present invention are for example obtained by lysing the cellular membrane of a microbial strain over-expressing at least one enzyme required for performing the desired reaction. For the purpose of the present invention, enzymes are considered as "over-expressed" when their expression is abnormal and excessively high compared to the non-modified strain, and the strain produces a pronounced gene-related phenotype. Typically, the microbial strain is modified with a plasmid comprising at least one gene encoding for the required enzyme.

In a particular aspect, the microbial strain from which the cell lysate is obtained is modified to over-express the enzymes required for the process of the present invention, as described above, and is further modified to inhibit or remove pathways that can compete with the reactions described above.

When one reaction requires several enzymes and such enzymes are provided as part of a cell-lysate, one microbial strain may over-express one, several, or all of such enzymes. If a strain does not over-express all genes required for the reaction, one or more additional lysate(s) from one or more other microbial strain(s) is(are) necessary or the missing enzymes can be added as isolated enzymes or as part of different matrices. In a particular aspect, each microbial strain over-expresses only one of the required enzymes.

In a particular aspect, the lysates are clarified. More preferably, the lysates are typically obtained by:

a) transforming the microbial strain with one or more plasmid(s) comprising one or more gene(s) encoding enzyme(s) intended to be over-expressed in the transformed strain;

b) lysing the cellular membrane of the microbial strain, such as to obtain a lysate enriched in the over-expressed enzyme(s) compared to a lysate of the non-transformed microbial strain; and

c) clarifying the lysate.

Microbial strain transformation, cell lysis and lysate clarification can be performed using any method commonly known by the person skilled in the art.

Lysate clarification is advantageous to avoid the consumption of intermediary products of the reactions involved in the present process by adjacent enzymes contained in the lysate. Indeed, the crude lysates derived from the microbial strain contain adjacent active pathways that can interfere with the conversion flow of the present reactions. By applying a clarification protocol, such enzymes can be removed without affecting the reactions of interest.

In another particular aspect, a cell lysate suitable as a source of enzyme for the purpose of the present invention has a total protein content of at least 10 mg/mL, based on the total volume of the cell lysate, as determined by the Bradford assay, which is well known to the person skilled in the art. In still another preferred aspect, such cell lysates have a content of each over-expressed enzyme of at least 1 mg/mL, based on the total volume of the cell lysate.

[Table 2] below provides a list of preferred enzymes used in the present invention, including for each enzyme the protein code and the protein name. In a preferred aspect, each enzyme preferably originates from the organism mentioned in the third column of the table. Each enzyme is further exemplified in one specific database entry of publication reference (fourth column).

[Table 2]

| Protein code | Protein name | Organism | Database ID |
|---|---|---|---|
| TDC | Tryptophan decarboxylase; Aromatic-L-amino acid decarboxylase | Catharanthus roseus | Uniprot: P17770 |
| PSI D | Tryptophan decarboxylase; Aromatic-L-amino acid decarboxylase | Psilocybe cubensis | Uniprot: P0DPA6 |
| AADC | Tryptophan decarboxylase; Aromatic-L-amino acid decarboxylase | Mus Musculus | Uniprot: O88533 |
| PSI H | Tryptamine 4-monooxygenase | Psilocybe cubensis | Uniprot: A0A286LF02 |
| CPR | Putative cytochrome P450 reductase | Psilocybe cubensis | Ref: ("Milne et al 2020) |
| PSI K | 4-hydroxytryptam ine kinase | Psilocybe cubensis | Uniprot: P0DPA8 |
| PSI M | N-methyltransferase | Psilocybe cubensis | Uniprot: P0DPA9 |
| SAMS | S-Adenosyl methionine synthetase | Escherichia coli | Uniprot: P0A817 |
| SAHH | S-adenosyl-l- | Psilocybe cubensis | NBCI: |
| | homocysteine hydrolase | | A0A650FP37 |
| ADOK | Adenosine kinase | Psilocybe cubensis | NBCI: A0A650FNZ7 |
| PPK2 class III | Polyphosphate kinase family 2 class III | Lampropedia hyalina DMS 16112 | NBCI: SHF67157.1 |
| PPK2 class I | Polyphosphate kinase family 2 class I | Sulfurovum lithotrophicum | NBCI: WP_ 046551064. 1 |
| PPK2 class III | Polyphosphate kinase family 2 class III | Erysipelotrichaceae bacterium | Uniprot: A0A3D5XRJ5 |

The process of the present invention also comprises the continuous recycling of the cofactors SAM and ATP (recycling valve). The present inventors have optimized the recycling valve in view of industrial cell-free enzymatic production of tryptamines. The SAM and ATP recycling valve used in the process of the present invention preferably consists of the four enzymes SAHH (EC:3.3.1.1), ADOK (EC:2.7.1.20), PPK2 class III (EC:2.7.4.1) and SAMS (EC:2.5.1.6) and two simple, inexpensive and easy to source substrates: polyphosphate and L-methionine.

The valve works first by transforming L-methionine by the action of the enzyme S-adenosyl methionine synthetase (SAMS) using one ATP molecule as energy cofactor for the reaction. To ensure that recycling of SAM until the reaction is substantially complete, L-methionine is preferably used in a L-methionine to L-tryptophan ratio of 2:1. SAM is then used in the main reaction as a methyl donor cofactor by the enzyme PsiM which transforms it into the molecule S-adenosyl-homocysteine (SAH). SAH is then separated into L-homocysteine and adenosine by the action of the enzyme homocysteine hydrolase (SAHH). L-homocysteine remains as a byproduct and adenosine enters the recycling route to become part of an ATP molecule once again. The process starts with the action of the enzyme adenosine kinase (AdoK) using ATP as a cosubstrate to generate adenosine monophosphate (AMP) and adenosine diphosphate (ADP). AMP is then converted into ADP by the action of the enzyme polyphosphate kinase from the enzyme family 2 and class III (PPK 2 class III) using as co-substrate inorganic polyphosphate to generate one ADP molecule. These newly formed ADP molecules are then transformed into ATP by the action of the same PPK 2 class III, optionally with polyphosphate kinase from the family 2 but from the class I (PPK 2 class I), using also inorganic polyphosphate as a sacrificial substrate for the reaction. Two examples of suitable PPK 2 class III enzymes are provided in Table 2. In a particular aspect of the invention, the PPK2 class III enzyme originates from Erysipelotrichaceae bacterium, and preferably it is the enzyme that bears the Uniprot reference A0A3D5XRJ5. Such PPK2 class III from Erysipelotrichaceae bacterium is particularly advantageous in that it can handle higher amount of polyphosphate than other PPK 2 class III enzymes, without loss of activity.

Polyphosphates are compounds consisting of several covalently linked phosphate units. In a preferred aspect

the polyphosphate consists of 3 to 10 phosphate units more preferably 4 to 7 phosphate units. Most preferably, the polyphosphate is polyP6 (i.e. a polyphosphate compounds consisting of 6 phosphate units).

Importantly, not all phosphate units of a polyphosphate compound are actually usable by for the phosphorylation of a substrate. The number of phosphate units that can be transferred to a substate are named as "available phosphate units". The number of available phosphate units depends on the number of phosphate groups in the polyphosphate compound and on the enzyme used to catalyze the phosphorylation. The number of available phosphate units for a given combination of polyphosphate compound and kinase can be determined by the person skilled in the art based on common general knowledge and simple experiments.

The amount of polyphosphate present in the reaction medium at the beginning of the process has to be sufficient, such that the number of available phosphate units is sufficient to recycle the cofactors until substantially all of the starting material is converted into the desired product. Therefore, the number of available phosphate units provided in the reaction medium shall be at least equal to n per molecule of L-tryptophan provided as starting material, wherein
n = 6 + [number of phosphate groups present in $R^1$ of the compound of Formula I].

When the process is for producing DMT, n = 6. All available phosphate units are involved in the recycling of the two SAM units required for the methylation of the amine. When the process is for producing psilocybin or psilocin, n=7. As for the production of DMT 6 phosphate units are required to recycle the two SAM units required for the methylation of the amine and one additional unit is used to recycle the ATP that acts as a donor of the phosphate in position R1 in psilocybin.

Preferably, the polyphosphate is polyP6. Depending on the kinase used, 2 to 4 phosphate groups of each polyP6 molecule are available for the enzyme. Therefore, to ensure that a sufficient number of phosphate units are available, it is preferred to provide at least 3.5 units of polyP6 in the reaction medium per molecule of L-tryptophan provided as starting material.

Preferably, the total amount of polyphosphate required for the reaction to continue until substantially all of the L-tryptophan is consumed is divided in several batches added at different time points during the reaction or polyphosphate is added continuously in the course of the reaction. This is advantageous to avoid inhibition of the kinase by an excessive concentration of polyphosphate in the reaction medium. More preferably, the addition rate is such that the concentration of polyphosphate in the reaction medium remains between 10 to 400mM, more preferably in a concentration of 50 to 400 mM, even more preferably 100 to 400 mM, most preferably 250 to 350 mM, most preferably this concentration is maintained throughout the reaction. In order to maintain such concentrations of polyphosphate, polyphosphate is re-injected when necessary, preferably in amounts such as to increase the concentration in the reaction medium by 20-100mM at every injection.

An initial pool of ATP has to be provided in the reaction medium. Such initial pool is preferably of 1 to 20 mM, preferably 5mM. This concentration is sufficient, as the present process provides for recycling of this cofactor.

In a preferred aspect of the invention, the process further comprises the recycling of NADP+ to NADPH. This is particularly useful when the tryptamine derivative of interest is either psilocin or psilocybin, as the production of these compounds involves NADPH as a cofactor. There have been enzymatic, chemical, electrochemical, and biological approaches to regenerate NADPH, but each has drawbacks in terms of cost, yield, ease of use, scalability, and efficacy for a variety of reasons. In a preferred aspect of the present process, the NADPH recycling valve comprises a Ni-Cu electrode, more preferably a nanostructured heterolayer of Ni-Cu2-O-Cu cathode that forms a photoelectrochemical process for direct electrochemical regeneration of NADPH from NADP+ using a mild overpotential (-0.75v). Sputtering of nickel on a copper oxide electrode provides an unexpectedly desirable surface morphology leading to high product selectivity. In an alternative preferred aspect, the recycling of NADPH is performed using citrate as a substrate. Citrate is converted into isocitrate by the action of the enzyme aconitate hydratase, preferably CAN (EC:4.2.1.3) and then isocitrate is converted into 2-oxoglutarate by the action of the enzyme isocitrate dehydrogenase, preferably IDH (EC:1.1.1.42), thus recycling NADP+ back to NADPH as a result of the enzymatic action.

Compared to the pathway previously described for *Psilocybe cubensis* in Demmler et al. 2020, the present recycling valve is advantageous in that it uses four enzymes instead of six, it consumes two ATP units instead of three, and it uses L-methionine as sacrificial substrate instead of 5-methyl-THF. The process performance is also optimized by the use of the GDP-polyphosphate kinase PPK2 class III, which performs two simultaneous different reactions, allowing for better conversion of ADP to ATP.

The use of a reduced number of enzymes advantageously goes with a simplification of the process. Indeed, enzymes often require specific conditions to exhibit their optimal catalytic activity and such conditions often differ from one enzyme to another characterized. Furthermore, enzymatic reactions may interfere with each other. Therefore, the higher the number of enzymes used in a process the more difficult it is to optimize the efficiency of the process.

The ATP saving is due to the replacement of the AdeK by PPK2 class III. This is advantageous because the saved ATP unit is made available for use in the mainstream process and the efficiency of the process is therefore improved.

The use of L-methionine as a sacrificial substrate instead of 5-methyl-tetrahdrofuran (5-methyl-THF) is advantageous, in that it avoids the accumulation of tetrahydrofuran (THF) as a by-product. Instead of THF, the present recycling

valve releases L-homocysteine as a by-product that accumulates during the course of the process. THF accumulation is more detrimental to the process efficiency than L-homocysteine accumulation, as THF interferes with catalytic activity of a diversity of enzymes. The accumulation of a by-product also makes it more complex to purify the tryptamine of interest at the end of the process. THF being a toxic and carcinogenic compound, residual amounts of THF in the final product would compromise the safety the produced tryptamines as a pharmaceutical product. To the contrary, homocysteine is an amino acid and traces of this by-product in the purified tryptamine of interest would not be harmful and would not prevent from using the obtained tryptamines for therapeutic purposes.

Additionally, with the use of polyphosphate as only phosphate source in the ATP recycling branch of the valve, the number of phosphate units present in the reaction medium can be controlled, such as to ensure that sufficient phosphate units are present to run the reaction until complete conversion of L-tryptophan to the tryptamine derivative of interest.

The invention further provides a reactor or a kit of reactors suitable for performing the process of the present invention. As explained above, the L-tryptophan decarboxylation, the double methylation of the amine and the optional substitution of a hydrogen atom on the benzene ring can be performed in one single reactor or in a kit of two or more reactors. When several steps are required to attach the desired substituent to the benzene ring, such steps can be all performed in one single reactor or be performed in two or more separate reactors.

A reactor (Reactor I) suitable for performing the L-tryptophan decarboxylation contains

a) L-tryptophan as the substrate; and

b) at least one amino acid decarboxylase, preferably an L-amino acid decarboxylase, more preferably an aromatic L-amino acid decarboxylase, most preferably selected from TDC, PsiD, AADC and mixtures thereof.

A reactor (Reactor II) suitable for performing the substitution of a hydrogen atom on the benzene ring such as to obtain a compound of Formula II wherein $R^1$ is a phosphate group or an -OH group contains

a) a monooxygenase, preferably the cytochrome P450-containing monooxygenase PsiH, together with a cytochrome P450 reductase (Cpr) which aids PsiH with electron transfer from NADPH,

b) a kinase, preferably the 4-hydroxytryptamine kinase PsiK;

c) the cofactors NADPH and ATP; and

d) an ATP recycling valve, comprising polyphosphate and a kinase, such as described above and

e) an NADPH recycling valve;

A reactor (Reactor III) suitable for performing the double methylation of the amine contains

a) at least one methyltransferase;

b) S-adenosyl methionine (SAM) as methyl donor;

c) A SAM recycling valve comprising

i) at least one hydrolase

ii) at least one kinase

iii) L-methionine

iv) ATP

v) a transferase capable of transferring an adenosyl group from ATP to L-methionine, preferably an S-adenosylmethionine synthase (SAMS); and

vi) polyphosphate in an amount sufficient to provide at least 6 available phosphate groups per molecule of L-tryptophan present in the reactor,

The methyltransferase is preferably PsiM when the desired compound of Formula I is psilocybin or psilocin and preferably INMT when the desired compound of Formula I is DMT.

Any two or more of reactors I to III can be merged in one single reactor. When all reactors are merged in one single reactor, the invention relates to a reactor. In all other cases, the invention relates to a kit of reactors. In a preferred aspect a single reactor is provided, wherein all reactions are performed in a one-pot process.

In the case of a single reactor, such reactor comprises:

a) L-tryptophan as the substrate;

b) at least one amino acid decarboxylase, preferably an L-amino acid decarboxylase, more preferably an aromatic L-amino acid decarboxylase, most preferably selected from TDC, PsiD, AADC and mixtures thereof;

c) optionally a monooxygenase, preferably the cytochrome P450-containing monooxygenase PsiH, together with a cytochrome P450 reductase (Cpr) which aids PsiH with electron transfer from NADPH,

d) at least one kinase, preferably at least two different kinases including a polyphosphate kinase and an adenosine kinase, and optionally a 4-hydroxytryptamine kinase PsiK;

e) optionally NADPH as a cofactor;

f) optionally an NADPH recycling valve, preferably such as described above;

g) ATP as a cofactor;

h) polyphosphate, preferably in a concentration of 10 to 400mM, more preferably in a concentration of 50 to 400 mM, even more preferably 100 to 400 mM, most preferably 300mM;

i) at least one methyltransferase;

j) S-adenosyl methionine (SAM) as methyl donor;

k) at least one hydrolase, preferably a S-adenosyl-l-homocysteine hydrolase;

l) L-methionine, preferably in a L-methionine to L-tryptophan ratio of at least 2:1, more of between 2:1 and 10:1;

m) a transferase capable of transferring an adenosyl group from ATP to L-methionine, preferably an S-adenosylmethionine synthetase (SAMS); and

n) polyphosphate in an amount sufficient to provide at least n available phosphate groups per molecule of L-tryptophan present in the reactor,

wherein n=6+[number of phosphate groups in $R^1$]

**Specific aspects of the invention**

**Process for producing psilocybin**

A method for producing a tryptamine derivative of Formula I

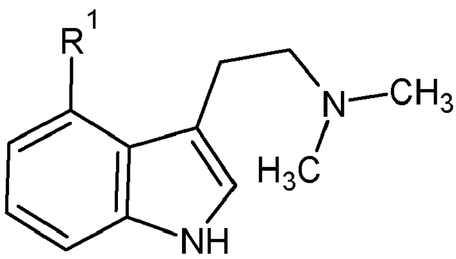

I

wherein $R^1$ is $-OPO(OH)_2$
by a cell-free biosynthesis process using L-tryptophan as a substrate, comprising

a) enzymatically converting L-tryptophan to tryptamine having the Formula II, wherein $R^1$ is hydrogen

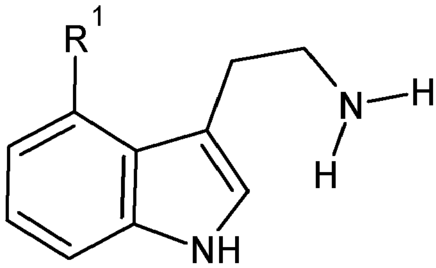

II

using at least one enzyme having an amino acid decarboxylase activity, preferably selected from AADC, TDC or PSI D, more preferably PSI D;
b) enzymatically substituting the hydrogen atom in position $R^1$ of tryptamine of Formula II with the radical $-OPO(OH)_2$ using the enzyme PSI H and PSI K and N and NADPH and ATP as donors;
c) enzymatically dimethylating the primary amine of a compound of Formula II as obtained in the end of step b), such as to produce a compound of Formula I, by using the enzyme PSI M and using two molecules of S-adenosyl methionine (SAM) as methyl donors, thus producing S-adenosyl cysteine (SAH) as a by-product;

wherein the method further comprises continuously recycling the donors SAM and ATP by

1) enzymatically cleaving the SAH produced in step c) into adenosine and L-homocysteine using a hydrolase, preferably the enzyme SAHH;

2) enzymatically phosphorylating adenosine using a kinase, preferably the enzyme AdoK, and adenosine triphosphate (ATP) as a phosphate donor, thus producing adenosine monophosphate (AMP) and adenosine diphosphate (ADP);

3) enzymatically phosphorylating the ADP and the AMP obtained in step 2) using a kinase and polyphosphate as a phosphate donor, such as to form ADP from AMP and ATP from ADP;

4) enzymatically phosphorylating the ADP produced in step 3) using a kinase and polyphosphate as a phosphate donor such at to form ATP;

5) enzymatically reacting L-methionine and ATP using an S-adenosyl methionine synthetase, preferably the enzyme SAMS, such as to generate SAM and tripolyphosphate;

wherein polyphosphate is provided during the process in an amount sufficient to provide at least 7 available phosphate groups per molecule of L-tryptophan used as starting material; and
wherein L-methionine is provided in an amount such that the L-methionine to L-tryptophan ratio is of at least 2:1.

**Reactor or kit of reactors for producing psilocybin**

A reactor or a kit of reactors for producing a compound of Formula I

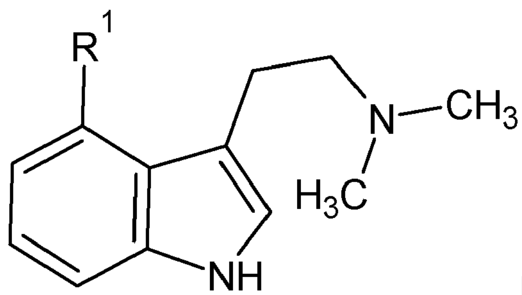

I

wherein R1 is $-OPO(OH)_2$;
comprising

1) a reactor I comprising

a) L-tryptophan;

b) at least one amino acid decarboxylase;

2) a reactor II comprising

a) at least one enzyme suitable for substituting a hydrogen atom in position $R^1$, of a compound of Formula II with the radical $-OPO(OH)_2$;

b) NADPH;

c) ATP

d) a recycling valve suitable to recycle NADPH; and

e) a recycling valve suitable to recycle ATP comprising at least one kinase and polyphosphate in an amount sufficient to provide at least 1 available phosphate group per molecule of L-tryptophan present in the reactor;

3) a reactor III comprising

a) at least one methyltransferase

b) S-adenosyl methionine (SAM)

c) A SAM recycling valve comprising

i) at least one hydrolase

ii) at least one kinase

iii) L-methionine

iv) ATP

v) a transferase capable of transferring an adenosyl group from ATP to L-methionine, preferably an S-adenosylmethionine synthase (SAMS); and

vi) polyphosphate in an amount sufficient to provide at least 6 available phosphate groups per molecule of L-tryptophan present in the reactor,

wherein any combination of two, or more of reactors I, II and III can be merged in one single reactor.

**Process for producing DMT**

A method for producing a tryptamine derivative of Formula I

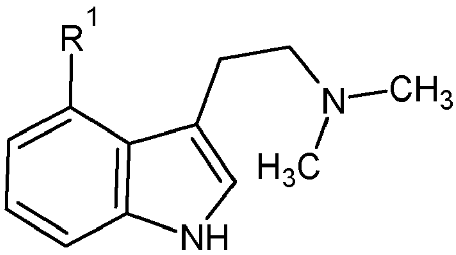

I

wherein R1 is -H;
by a cell-free biosynthesis process using L-tryptophan as a substrate, comprising

a) enzymatically converting L-tryptophan to tryptamine having the Formula II, wherein $R^1$ is hydrogen

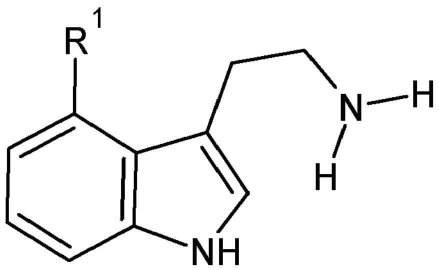

II

using at least one enzyme having an amino acid decarboxylase activity, preferably selected from AADC, TDC or PSI D, more preferably AADC;

b) enzymatically dimethylating the primary amine of a compound of Formula II as obtained in the end of step a), such as to produce a compound of Formula I, by using at least one methyltransferase and using two molecules of S-adenosyl methionine (SAM) as methyl donors, thus producing S-adenosyl cysteine (SAH) as a by-product;

wherein the method further comprises continuously recycling the methyl donor SAM by

1) enzymatically cleaving the SAH produced in step c) into adenosine and L-homocysteine using a hydrolase, preferably the enzyme SAHH;

2) enzymatically phosphorylating adenosine using a kinase, preferably the enzyme AdoK, and adenosine triphosphate (ATP) as a phosphate donor, thus producing adenosine monophosphate (AMP) and adenosine diphosphate (ADP);

3) enzymatically phosphorylating the ADP and the AMP obtained in step 2) using a kinase and polyphosphate as a phosphate donor, such as to form ADP from AMP and ATP from ADP;

4) enzymatically phosphorylating the ADP produced in step 3) using a kinase and polyphosphate as a phosphate donor such at to form ATP;

5) enzymatically reacting L-methionine and ATP using a transferase, preferably an S-adenosyl methionine synthetase, preferably the enzyme SAMS, such as to generate SAM and tripolyphosphate;

wherein polyphosphate is provided during the process in an amount sufficient to provide at least 6 available phosphate groups per molecule of L-tryptophan used as starting material; and
wherein L-methionine is provided in an amount such that the L-methionine to L-tryptophan ratio is of at least 2:1.

**Reactor or kit of reactors for producing DMT**

A reactor or a kit of reactors for producing a compound of Formula I

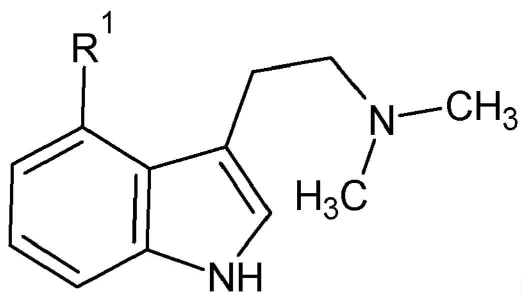

I

wherein R1 is -H;
comprising

1) a reactor I comprising

a) L-tryptophan;
b) at least one amino acid decarboxylase;

2) a reactor III comprising

a) at least one methyltransferase

b) S-adenosyl methionine (SAM) as methyl donor,

c) A SAM recycling valve comprising

i) at least one hydrolase

ii) at least one kinase

iii) L-methionine

iv) ATP

v) a transferase capable of transferring an adenosyl group from ATP to L-methionine, preferably an S-adenosylmethionine synthase (SAMS); and

vi) polyphosphate in an amount sufficient to provide at least 6 available phosphate groups per molecule of L-tryptophan present in the reactor,

wherein any combination of two, or more of reactors I, II and III can be merged in one single reactor.

## Examples

### Example 1: Computational Kinetic Modeling of Enzymatic Synthesis of psilocybin

1. Introduction

Biochemical networks are intrinsically complex, not only because they include a large number of interacting components, but also because those interactions are nonlinear. Like many other nonlinear phenomena in nature, their behavior is not intuitive and thus quantitative models are required to describe and understand their function. From a model perspective, biochemical networks are a set of chemical species that can be converted into each other through chemical reactions. The focus of biochemical network models is usually on the levels of the chemical species and this usually requires explicit mathematical expression for the velocity at which the reaction proceed. Dynamics are introduced in models through the kinetic of the molecular interactions, the majority of which are enzyme-catalyzed reactions. The determination of kinetic parameters and rate laws is thus an important activity in systems biology. Traditionally enzyme kinetic is the vehicle for determining reaction mechanisms. Therefore, to construct biochemical networks models it is important to determine the kinetic properties of the enzymes in conditions as close as possible to the cellular environment. One of the most well-known mathematical model that describes the enzymatic activity by calculating the relative kinetic parameters is the Michaelis-Menten (MM) kinetic model.

2. The Michaelis-Menten (MM) kinetic model

In biochemistry one of the best-known models for the enzymatic kinetics is the Michaelis-Menten (MM) kinetics (see Nelson et al.). The model assumes that an enzyme E binds a substrate S to form a complex ES, which releases a product P regenerating the original enzyme:

[Equ. 1]

$$E + S \underset{k_{-1}}{\overset{k_1}{\rightleftharpoons}} ES \underset{k_{-2}}{\overset{k_2}{\rightleftharpoons}} E + P$$

The double arrows between S and ES represent the fact that enzyme-substrate binding is a reversible process, and the single forward arrow represents the irreversible formation of the product. $k_1$ is the rate constant of the formation of ES, $k_{-1}$ the rate constant of the dissociation of the complex ES reforming the substrate S, and $k_2$ (also known as Kcat, catalytic rate constant) is the rate constant for the formation of the product P.

Assuming that ES is in rapid equilibrium with S and that the formation of P is irreversible ($\Delta G << 0$), the model can be synthesized in an equation that describes the rate of the enzymatic reaction (v, i.e., the rate of formation of the product P) in function of the concentration of the substate S. This relation can be written as:

[Equ. 2]

$$v = d[P]/dt = (V_{max}*[S])/(K_m*[S])$$

The equation is called Michaelis-Menten equation, and it is the basic equation for enzyme kinetics. $V_{max}$ (expressed in $M^{-1}s^{-1}$) represents the maximum rate that can be achieved by the system (i.e., how many moles of substrates are converted in product in one second), and $K_m$ (expressed as a concentration, mol $L^{-1}$), defined as the Michaelis-Menten constant, is numerically equal to the substrate concentration at which the reaction rate is half of $V_{max}$. Km has a different value for each enzyme-substrate couple, and it is a measure of the affinity between an enzyme and a specific substrate. Indeed, if an enzyme has a low value of $K_m$, this means that it achieves maximal catalytic efficiency at low substrate concentrations, so that it has more affinity for that substrate. See [Fig. 3].

Another useful parameter to characterize the activity of an enzyme is the catalytic efficiency. According to the MM kinetic model, the catalytic constant $K_{cat}$, i.e., the rate constant for the formation of the product P from the complex EX, can be defined as:

[Equ. 3]

$$Kcat = Vmax/[E]$$

where [E] is the initial concentration of the enzyme, which is not supposed to change during the process. $K_{cat}$ is also known as turnover number, because it is the number of reaction processes (turnovers) that each active size catalyzes per unit time. Ideally, we can assume that [E] is constant over the time.

MM model provides the equations that allows to calculate how fast the products of an enzymatic process are being formed. Enzyme catalyzed reaction kinetic data ($K_m$, $V_{max}$, $K_{cat}$, etc.) are stored in online databases (such as BRENDA (see Pharkya et al. 2003 and Schomburg et al. 2002) or SABIO-K (see Wittig et al. 2012)) or in published research and can be consulted and downloaded. If we consider a biochemical network as sets of reactions that are lined each other by common substrates and products, and if we set a MM rate law for every reaction of the network, the dynamics of the biochemical network is frequently described as a set of coupled ordinary differential equations (ODEs) that represents the rate of change of concentrations of the chemical species involved in the network. The right-hand side of these ODEs is the algebraic sum of the rate laws of the reactions that produce or consume the species (positive when it is produced, negative when it is consumed). There are several software packages that solve numerically these ODEs, thus modeling biochemical networks. Among all these software, we have chosen COPASI, one of the most used and cited in literature.

3. Computational Modeling of Biochemical Networks Using COPASI

COPASI (COmplex PAthway Simulator) is an open-source software application for creating and solving mathematical models of biological processes (see Hoops et al. 2006, Mendes et al 2009 and Proceedings of the 38th conference on Winter simulation). It contains algorithms for simulation through ODEs, algebraic equations, derivatives and discrete events. It also allows to mix several of these in a single simulation. The COPASI user represents a biochemical network model through the language of the biochemistry, while the software internally constructs the appropriate mathematical representations. COPASI supports several integrated rate laws (the user can also implement his own rate law), including the MM kinetic law. To build a biochemical network in COPASI the following features are required:

1) Compartment: whether extracellular, membranous, cytoplasmatic, etc.

2) Species: whether substrates, products, modifiers, etc.

3) Parameters : $V_{max}$, $K_m$, $K_{cat}$, etc.

4) Unit definition: concentrations, time, etc.

5) Reactions: whether reversible or irreversible.

6) Function definition: individual reaction rate laws to be followed during the pathway simulations, such as MM, allosteric activation, competitive inhibition and many others.

7) Initial assignment: initial concentrations of enzymes and substrates, whether a specie is fixed or change its concentration over the time, duration of the simulations, etc.

8) Constraints: to be followed during the simulations.

4. Computational Kinetic Study of Enzymatic Synthesis of Psilocybin

Here we show the computational kinetic study by means of COPASI of the enzymatic synthesis of psilocybin, To do that, it is fundamental to understand how the different concentrations of substrates and enzymes, as well as how the consumption of the cofactors involved in the biochemical synthesis, limit the yield. In this sense, the computational models are crucial to set up the best concentrations of all the species, the best thermodynamical conditions and implement further biochemical channels that lead to the regeneration of the enzymatic cofactors.

Biosynthetically, the biochemical transformation from tryptophan to psilocybin involves five enzyme reactions: decarboxylation, double methylation at the N9 position, 4-hydroxylation, and O-phosphorylation. One sequence of the intermediate enzymatic steps has been shown to involve 4 different enzymes (PsiD, PsiH, PsiK, and PsiM) and three

different cofactors (ATP, NADPH and SAM). Our goal was to simulate the entire biological process, starting from experimental data, in order to analyze these different features:

- How the initial concentrations of enzymes, substrates and cofactors affect the rate and the final yield. Find the best thermodynamic parameters for the maximal final yield.
- How much the consumption of the cofactors slows down the process.

To do that, we have implemented in COPASI the 5 reactions that compose the biological network.

1) L-tryptophane $\xrightarrow{\text{PSI-D}}$ Tryptamine

2) Tryptamine + NADPH $\xrightarrow{\text{PSI-H}}$ 4-hydroxytryptamine

3) 4-hydroxytryptamine + ATP $\xrightarrow{\text{PSI-K}}$ Norbaeocystein

4) Norbaeocystein + SAM $\xrightarrow{\text{PSI-M}}$ Baeocystein

5) Baeocystein + SAM $\xrightarrow{\text{PSI-M}}$ Psilocybin

All the reactions were considered irreversible and for each of them a MM rate law was specified. The reactions (2) to (5) involve also other species as substrates (NADPH, ATP and SAM), which can be considered as "helper molecules" that assist in biochemical transformations, and their consumption must be included in the rate laws. In the case of reaction (1), no cofactor is involved in the enzymatic catalytic activity, so the rate law implemented is equal to [Equ. 2]. Conversely, when a cofactor is involved, the reaction was considered as a non-interacting bireactant enzymes reaction, and the relative rate law can be written as:

[Equ. 4]

$$V = (V_{max}*[S]*[Cof])/((K_m_S*K_m_Cof) + (K_m_S*[Cof]) + (K_m_S*[S]) + ([S]*[Cof]))$$

where [Cof] is the concentration of the cofactor, and $K_m$_S and $K_m$_Cof are the MM constants for the enzymatic reaction on the substrate and on the cofactor respectively. Since the mechanisms are concerted, $K_m$_S and $K_m$_Cof were considered equal. The input parameters that were implemented, Km, $V_{max}$, [S] and [Cof], are summarized in [Table 3] and [Table 4]. If the values of $V_{max}$ cannot be specified because not available, COPASI set them at 0.1 mol/L by default.

[Table 3]: enzymatic parameters used for the COPASI computational study of the enzymatic synthesis of psilocybin

| Protein code | Protein name | Organism | Database ID | Enzyme kinetics |
|---|---|---|---|---|
| PSI D | Tryptophan decarboxylase; Aromatic-L-amino acid decarboxylase | Psilocybe cubensis | Uniprot: P0DPA6 | $K_M$= 0.729mM *** values from Capsicum annuum |
| PSI H | Tryptamine 4-monooxygenase | Psilocybe cubensis | Uniprot: A0A286LF02 | $K_M$= 0.22mM |
| PSI K | 4-hydroxytryptam ine kinase | Psilocybe cubensis | Uniprot: P0DPA8 | $K_M$= 0.08mM |
| PSI M | N-methyltransferase | Psilocybe cubensis | Uniprot: P0DPA9 | $K_M$= 0.165mM |

[Table 2]: initial concentrations of the initial substrate (L-tryptophan) and the cofactors ised for the COPASI computational study of the enzymatic synthesis of psilocybin

| | **Initial concentration** |
|---|---|
| L-tryptophan | 1 mM - 1 M, preferably 200mM |
| ATP | 1 mM - 1 M, preferably 6mM |
| NADPH | 1 mM - 1 M, preferably 6mM |
| SAM | 1 mM - 1 M, preferably 6mM |

5. Results and Discussion

In [Fig. 4] and [Fig. 5] are reported the time course evolutions of the concentrations of the different species in the enzymatic synthesis of psilocybin. On the x axis is reported the time (s) while on y axis is reported the concentration (mol/L).

From [Fig. 4], it can be seen that after 2s the initial substrate (L-tryptophan) is completely consumed. The formation of tryptamine is specular to the curve of L tryptophan, since no cofactor limits the formation of tryptamine. As soon as the concentration of L-tryptophan reached zero, no more formation of tryptamine was observed. [Fig. 5] is a zoom on the lower part of the graph of [Fig. 4] and shows that other species are formed, namely psilocybin. However, the formation of such species is influenced by the consumption of cofactors and the formation of such species, stopped as soon as the concentration of the necessary cofactors reached zero. Among all the cofactors, SAM was consumed first since it is involved in two consecutive reactions (reactions 4 and 5). The curves of the decrease of the concentrations of NADPH and ATP are almost overlapped. The formation of the final product (psilocybin) was not observed over 0.6 seconds, which coincides with the total consumption of the cofactor SAM. As soon as SAM is consumed, reactions 4 and 5 cannot occur, as well as reactions 2 and 3 when NADPH and ATP are consumed, respectively.

These results demonstrate that recycling of all cofactors is required to ensure that substantially the whole amount of starting material is converted to the desired product and to avoid that the reactions stops at the level of an intermediate product, thus compromising the psilocybin yield.

**Citation List**

Non-Patent Literature Citation List

Aday, Jacob S., Mitzkovitz, Cayla M., Bloesch, Emily K., Davoli, Christopher C., and Davis, Alan K. 2020. "Long-Term Effects of Psychedelic Drugs: A Systematic Review" Neuroscience and Biobehavioral Reviews 113 (June): 179-89 (cited herein as Aday et al. 2020)

Nutt, David, Erritzoe, David, and Carhart-Harris, Robin. 2020. "Psychedelic Psychiatry’s Brave New World." Cell . https://doi.org/10.1016/j.cell.2020.03.020 (cited herein as Nutt et al. 2020).

Fricke, Janis, Blei, Felix, and Hoffmeister, Dirk. 2017. "Enzymatic Synthesis of Psilocybin." Angewandte Chemie 56 (40): 12352-55 (cited herein as Fricke et al. 2017).

Fricke, Janis, Kargbo, Robert, Regestein, Lars, Lenz, Claudius, Peschel, Gundela, Rosenbaum, Miriam A., Sherwood, Alexander, and. 2020. "Scalable Hybrid Synthetic/Biocatalytic Route to Psilocybin". Chem. Eur. J. 26: 8281-8285 (cited herein as Fricke et al. 2020).

Demmler, Richard, Fricke, Janis, Dörner, Sebastian, Gressler, Markus, and Hoffmeister, Dirk. 2020. "S-Adenosyl-L-Methionine Salvage Impacts Psilocybin Formation in ’Magic’ Mushrooms." Chembiochem: A European Journal of Chemical Biology 21 (9): 1364-71 (cited herein as Demmler et al. 2020).

Dean, Jon G. 2018. "Indolethylamine-N-Methyltransferase Polymorphisms: Genetic and Biochemical Approaches for Study of Endogenous N,N,-Dimethyltryptamine." Frontiers in Neuroscience https://doi.org/10.3389/fnins.2018.00232 (cited herein as Dean 2018).

Nelson, D. L. (David L.; Cox, M. M.; Hoskins, A. A.; Lehninger, A. L. Lehninger Principles of Biochemistry. 3 (cited herein as Nelson et al.).

Pharkya, P.; Nikolaev, E. v.; Maranas, C. D. Review of the BRENDA Database. Metabolic engineering 2003, 5 (2), 71-73 (cited herein as Pharkya et al. 2003).

Schomburg, I.; Chang, A.; Schomburg, D. BRENDA, Enzyme Data and Metabolic Information. Nucleic Acids Research 2002, 30 (1), 47 (cited herein as Schomburg et al. 2002).

Wittig, U.; Kania, R.; Golebiewski, M.; Rey, M.; Shi, L.; Jong, L.; Algaa, E.; Weidemann, A.; Sauer Danzwith, H.; Mir, S.; Krebs, O.; Bittkowski, M.; Wetsch, E.; Rojas, I.; Muller, W. SABIO-RK-Database for Biochemical Reaction Kinetics. Nucleic Acids Research 2012, 40 (Database issue), D790 (cited herein as Wittig et al. 2012).

Hoops, S.; Gauges, R.; Lee, C.; Pahle, J.; Simus, N.; Singhal, M.; Xu, L.; Mendes, P.; Kummer, U. COPASI-a COmplex PAthway Simulator. Bioinformatics 2006, 22 (24), 3067-3074 (cited herein as Hoops et al. 2006).

Mendes, P.; Hoops, S.; Sahle, S.; Gauges, R.; Dada, J.; Kummer, U. Computational Modeling of Biochemical Networks Using COPASI. Methods in Molecular Biology 2009, 500 (1), 17-59 (cited herein as Mendes et al. 2009).

Simulation of biochemical networks using COPASI | Proceedings of the 38th conference on Winter simulation https://dl.acm.org/doi/10.5555/1218112.1218421 (accessed 2022-01-21) (cited herein as Proceedings of the 38th conference on Winter simulation)

## Claims

**1.** A method for producing a tryptamine derivative of Formula I

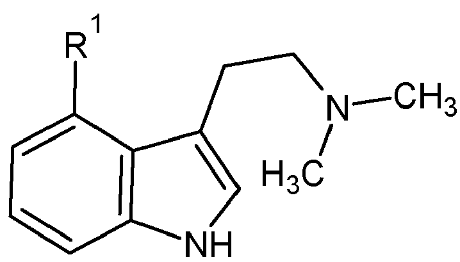

I

wherein $R^1$ is selected from -H, $-OPO(OH)_2$, and -OH;

and wherein the tryptamine derivative of Formula I is selected from the group consisting of psilocybin, psilocin and N,N-dimethyltryptamine (DMT);
by a cell-free biosynthesis process using L-tryptophan as a substrate, comprising

a) enzymatically converting L-tryptophan to tryptamine having the Formula II, wherein $R^1$ is hydrogen

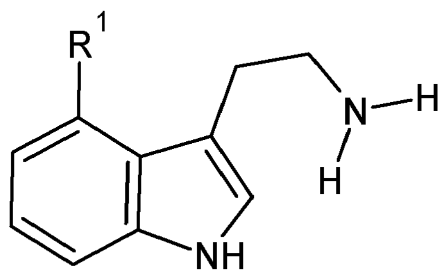

II

using at least one enzyme having an amino acid decarboxylase activity;
b) optionally enzymatically substituting the hydrogen atom in position $R^1$ of tryptamine of Formula II with a

radical selected from -$OPO(OH)_2$, and -OH using at least one suitable enzyme and at least one suitable donor,

c) enzymatically dimethylating the primary amine of a compound of Formula II as obtained in the end of step a) or step b), such as to produce a compound of Formula I, by using at least one methyltransferase and using two molecules of S-adenosyl methionine (SAM) as methyl donors, thus producing S-adenosyl cysteine (SAH) as a by-product;

wherein the method further comprises continuously recycling the methyl donor SAM and the cofactor ATP by

1) enzymatically cleaving the SAH produced in step c) into adenosine and L-homocysteine using a hydrolase, preferably the enzyme SAHH;
2) enzymatically phosphorylating adenosine using a kinase, preferably the enzyme AdoK, and adenosine triphosphate (ATP) as a phosphate donor, thus producing adenosine monophosphate (AMP) and adenosine diphosphate (ADP);
3) enzymatically phosphorylating the ADP and the AMP obtained in step 2) using a kinase and polyphosphate as a phosphate donor, such as to form ADP from AMP and ATP from ADP;
4) enzymatically phosphorylating the ADP produced in step 3) using a kinase and polyphosphate as a phosphate donor such at to form ATP;
5) enzymatically reacting L-methionine and ATP using a transferase, preferably a S-adenosyl methionine synthetase, preferably the enzyme SAMS, such as to generate SAM and tripolyphosphate;

wherein polyphosphate is provided during the process in an amount sufficient to provide at least n available phosphate groups per molecule of L-tryptophan provided as starting material,

wherein n= 6 + [number of phosphate groups present in $R^1$ of the compound of Formula I]; and
wherein L-methionine is provided in an amount such that the L-methionine to L-tryptophan ratio is of at least 2:1.

**2.** A method according to claim 1, wherein the enzyme having an amino acid decarboxylase activity is selected from the group consisting of AADC, PSID, TDC and combinations thereof.

**3.** A method according to claim 1 or 2, wherein the kinase is PPK2 class III kinase, optionally together with a PPK2 class I kinase

**4.** A method according to claim any one of claims 1 to 3, wherein the tryptamine derivative of Formula I is psilocybin,

step b) comprises enzymatically converting tryptamine of Formula II to 4-hydroxytryptamine using
a monooxygenase, preferably the cytochrome P540-containing monooxygenase PSI H and a cytochrome P450 reductase,
and using NADPH as cofactor and
enzymatically converting 4-hydroxytryptamine to norbaeocystin using a kinase, preferably PSI K, and ATP as an energy cofactor;
wherein step c) comprises enzymatically dimethylating the primary amine of norbaeocystin such as to form psilocybin using PSI M;
and wherein n=7.

**5.** A method according to any one of claims 1 to 3, wherein the tryptamine derivative of Formula I is psilocin, wherein steps a) to c) are performed as described in claim 4, such as to produce psilocybin
and wherein the process further comprises a step of dephosphorylating psilocybin by placing psilocybin in a neutral to acidic environment or by performing an enzymatic dephosphorylation.

**6.** A method according to any of the preceding claims, wherein the method further comprises continuously recycling $NADP^+$ to NADPH.

**7.** A method according to claim 6, wherein the continuous recycling of NADP+ to NADPH is performed using a Ni-Cu electrode or a recycling valve wherein citrate is used as a sacrificial substrate which is transformed to isocitrate by an aconitate hydratase and isocitrate is converted to 2-oxoglutarate by an isocitrate dehydrogenase using NADP+ as a co-substrate, thus converting NADP+ to NADPH.

**8.** A method according to any one of claims 1 to 3, wherein the tryptamine derivative of Formula I is N,N-dimethyltryptamine,

wherein optional step b) is not performed,
wherein step c) comprises enzymatically converting tryptamine to dimethyltryptamine using the methyl transferase INMT;
and wherein n = 6.

**9.** A reactor or a kit of reactors for producing a compound of Formula I

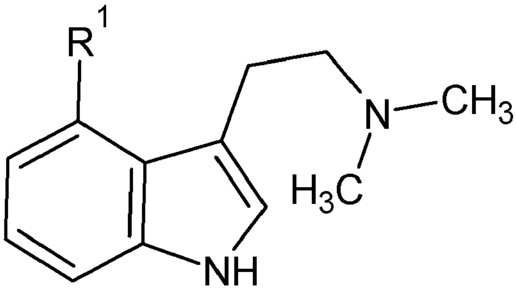

I

wherein $R^1$ is selected from -H, $-OPO(OH)_2$, and -OH;

and wherein the tryptamine derivative of Formula I is selected from the group consisting of psilocybin, psilocin and N,N-dimethyltryptamine (DMT);
comprising

1) a reactor I comprising

a) L-tryptophan;
b) at least one amino acid decarboxylase;

2) optionally a reactor II comprising

a) at least one enzyme suitable for substituting a hydrogen atom in position $R^1$ of a compound of Formula II with another radical selected from -OHPO3- and -OH;

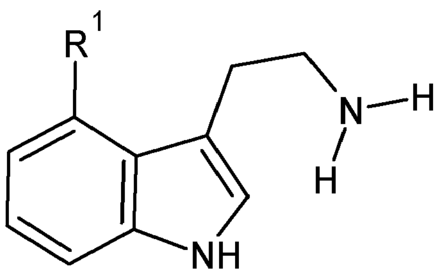

II

b) at least one donor suitable for forming a radical selected from $-OPO(OH)_2$ or -OH; and
c) a recycling valve suitable to recycle the at least one donor;

3) a reactor III comprising

a) at least one methyltransferase
b) S-adenosyl methionine (SAM) as methyl donor,
c) A SAM recycling valve comprising

i) at least one hydrolase, preferably a S-adenosyl homocysteine hydrolase,
ii) at least one kinase, preferably a polyphosphate kinase and an adenosine kinase,

iii) L-methionine
iv) ATP
v) a transferase capable of transferring an adenosyl group from ATP to L-methionine, preferably an S-adenosylmethionine synthase (SAMS); and
vi) polyphosphate,

wherein any combination of two or more of reactors I, II and III can be merged in one single reactor.

**10.** A reactor or a kit of reactors according to claim 9, wherein the at least one amino acid decarboxylase is as described in claim 2 and wherein the polyphosphate kinase is as described in claim 3.

**11.** A reactor according to claim 9 or 10, wherein ATP is present in an amount of 1 to 20 mM or a kit of reactors according to claim 9 or 10 wherein ATP is present in an amount of 1 to 20 mM in each of reactor II and reactor III.

**12.** A reactor or a kit of reactors according to any one of claims 9 to 11, wherein the tryptamine derivative of Formula I is psilocybin or psilocin, wherein the at least one enzyme suitable for substituting a hydrogen atom in position $R^1$ with another radical is a monooxygenase, preferably the cytochrome P540-containing monooxygenase PSI H, together with a cytochrome P450 reductase (Cpr) and a kinase, preferably PSI K;

wherein reactor II contains two donors which are NADPH and ATP;
wherein reactor II comprises two recycling valves for recycling the donors which consist in

- a NADPH recycling valve and
- an ATP recycling valve comprising polyphosphate and a kinase; and

wherein the at least one methyltransferase in reactor III is PSI M.

**13.** A reactor or a kit of reactors according to claim 12, wherein the recycling valve for recycling NADP+ to NADPH is selected from

a) a Ni-Cu electrode or
b) a recycling valve comprising citrate, an aconitate hydratase and an isocitrate dehydrogenase.

**14.** A reactor or a kit of reactors according to any one of claims 9 to 11, wherein the tryptamine derivative of formula I is DMT,

wherein the optional reactor II is not present; and
wherein the at least one methyltransferase in reactor III is INMT; and

**15.** A reactor or a kit of reactors according to any one of claims 9 to 14, wherein polyphosphate, where present, is in a concentration of 10 to 400 mM, preferably in a concentration of 50 to 400 mM.

[Fig. 1]

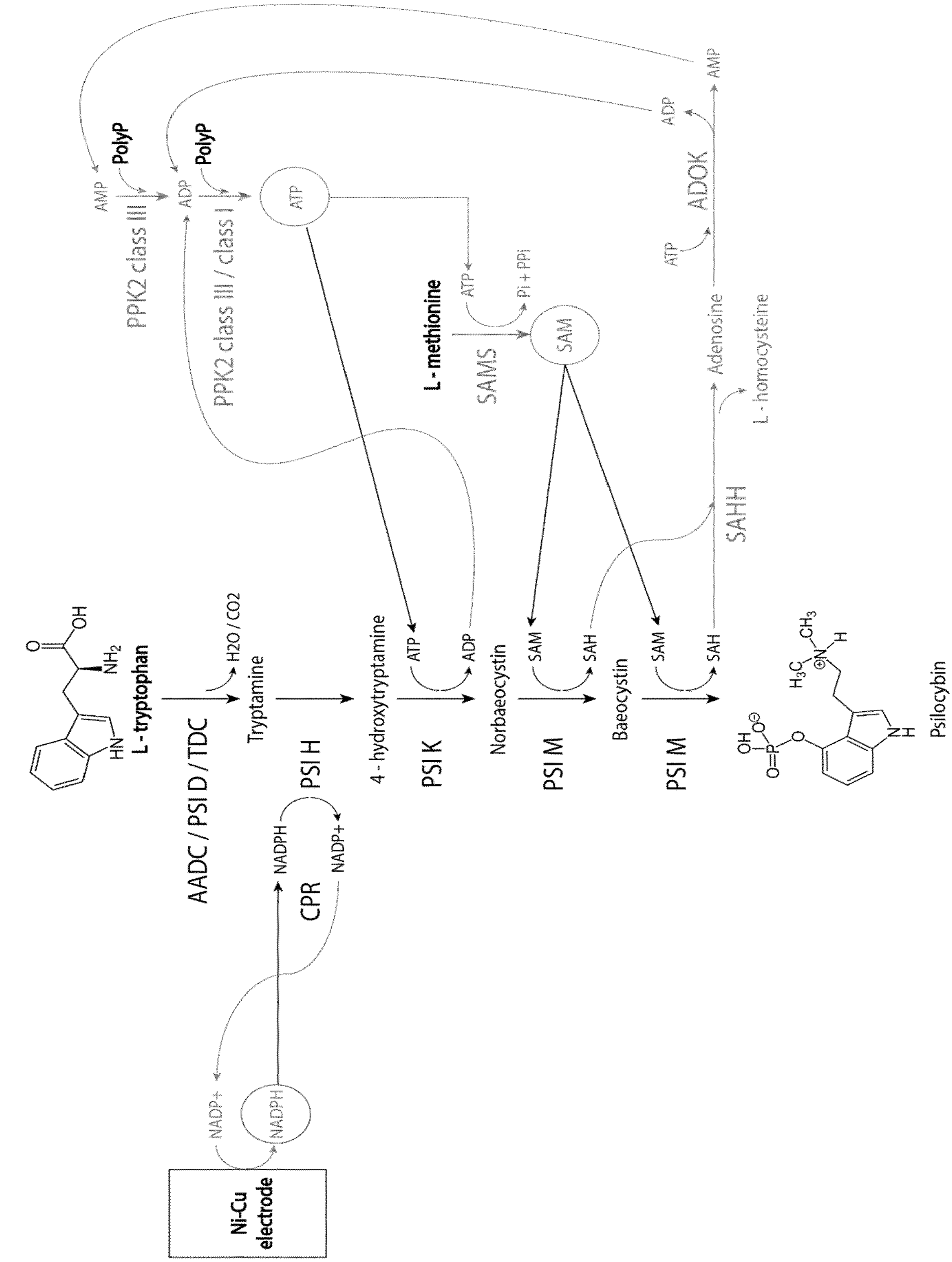
L - tryptophan
AADC / PSI D / TDC
H2O / CO2
Tryptamine
PSI H
NADPH
NADP+
CPR
4 - hydroxytryptamine
PSI K
ATP
ADP
Norbaeocystin
PSI M
SAM
SAH
Baeocystin
PSI M
SAM
SAH
Psilocybin
Ni-Cu electrode
NADPH
NADP+
PPK2 class III
PPK2 class III / class I
AMP
ADP
PolyP
PolyP
ATP
L - methionine
SAMS
ATP
Pi + PPi
SAM
SAHH
Adenosine
L - homocysteine
ATP
ADOK
ADP
AMP

[Fig. 2]

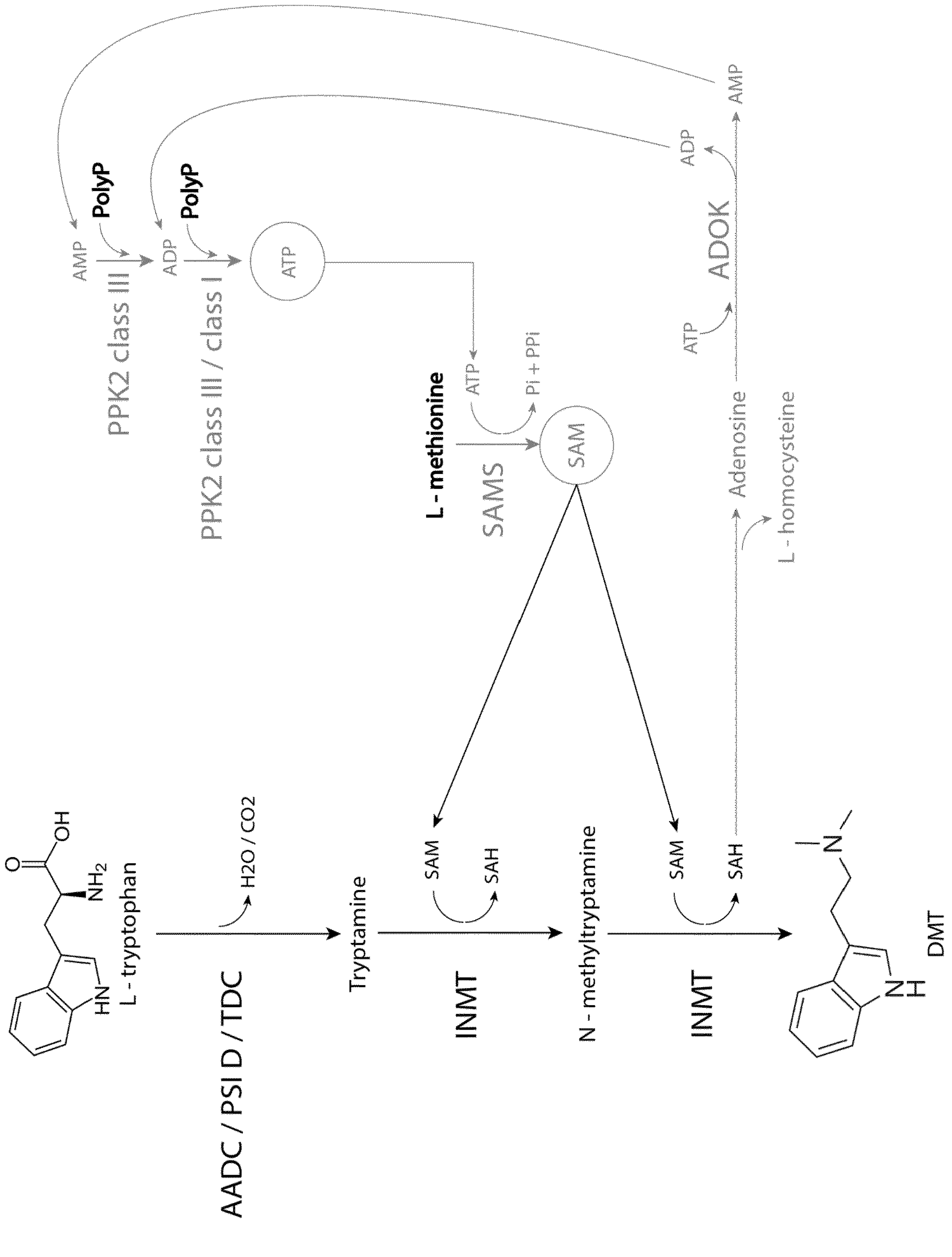

L - tryptophan
AADC / PSI D / TDC
H2O / CO2
Tryptamine
INMT
SAM
SAH
N - methyltryptamine
INMT
SAM
SAH
DMT
L - methionine
SAMS
ATP
Pi + PPi
SAM
PPK2 class III
PPK2 class III / class I
AMP
PolyP
ADP
PolyP
ATP
Adenosine
L - homocysteine
ATP
ADOK
ADP
AMP

[Fig. 3]

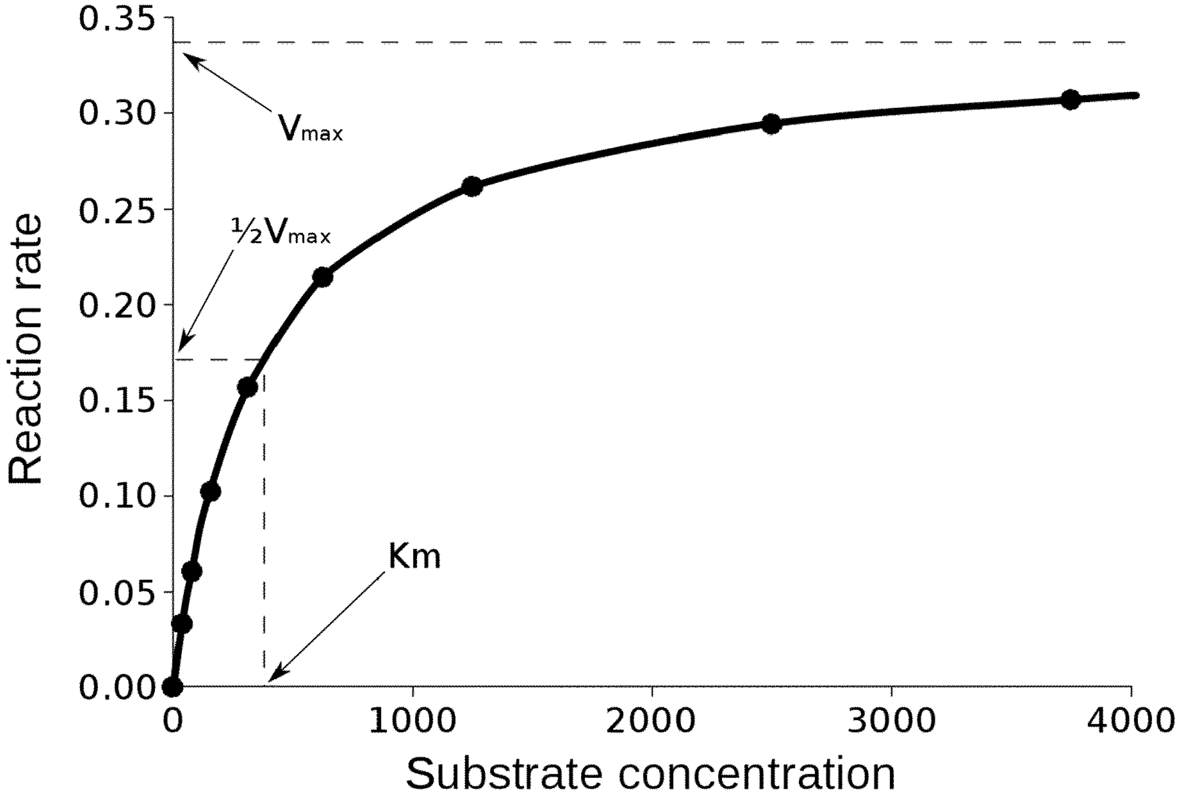
0.35
0.30
0.25
0.20
0.15
0.10
0.05
0.00
Reaction rate
0
1000
2000
3000
4000
Substrate concentration
Vmax
½Vmax
Km

[Fig. 4]

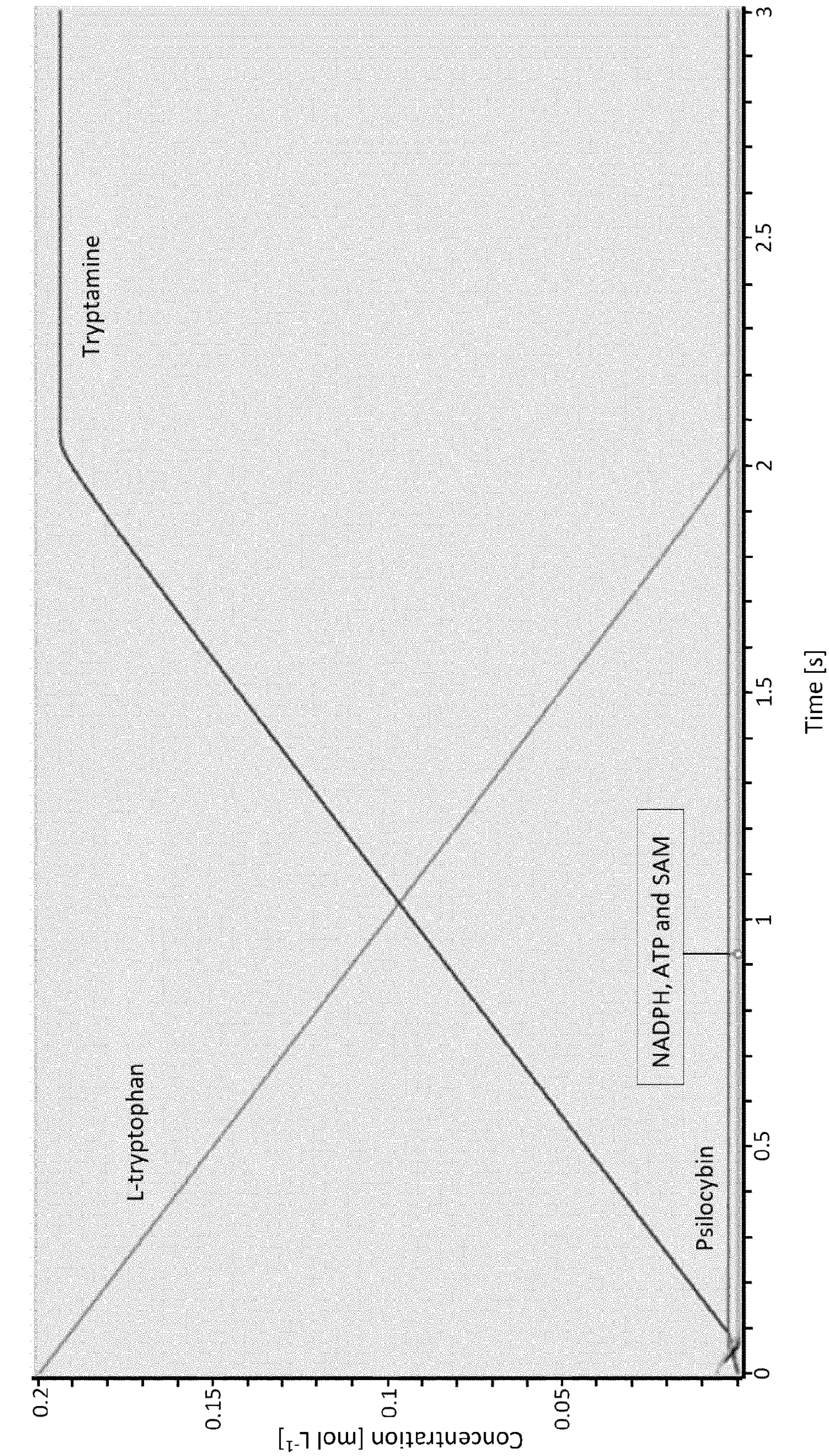
Tryptamine
L-tryptophan
Psilocybin
NADPH, ATP and SAM
Time [s]
0
0.5
1
1.5
2
2.5
3
Concentration [mol L-1]
0.2
0.15
0.1
0.05

[Fig. 5]

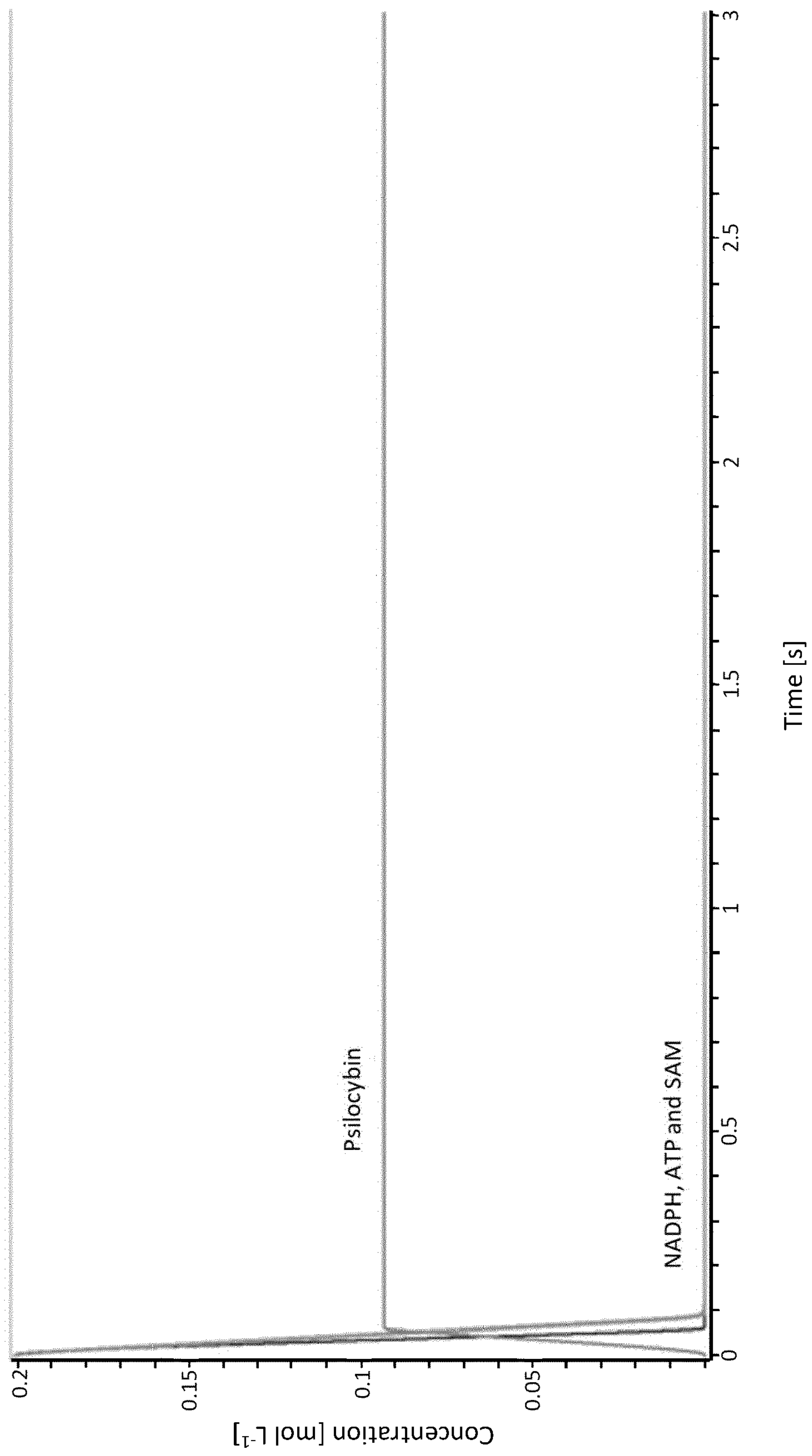
Psilocybin
NADPH, ATP and SAM
Time [s]
Concentration [mol L-1]
0
0.5
1
1.5
2
2.5
3
0.05
0.1
0.15
0.2

Europäisches Patentamt
European Patent Office
Office européen des brevets

# EUROPEAN SEARCH REPORT

Application Number
EP 22 15 4810

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2019/180309 A1 (TEKNOLOGIAN TUTKIMUSKESKUS VTT OY [FI]) 26 September 2019 (2019-09-26) * the whole document * ----- | 1-15 | INV. C12P17/10 A61K31/675 A61P25/00 C07D209/16 C12N9/02 C12N9/10 C12N9/12 C12N9/88 |
| Y,D | DEMMLER RICHARD ET AL: "-Methionine Salvage Impacts Psilocybin Formation in "Magic" Mushrooms", CHEMBIOCHEM, vol. 21, no. 9, 10 January 2020 (2020-01-10), pages 1364-1371, XP055945208, ISSN: 1439-4227, DOI: 10.1002/cbic.201900649 Retrieved from the Internet: URL:https://onlinelibrary.wiley.com/doi/full-xml/10.1002/cbic.201900649> * the whole document * ----- | 1-15 | |
| Y | MICKLEFIELD ED - MICKLEFIELD: "Streamlined recycling of S-adenosylmethionine", NATURE CATALYSIS,, vol. 2, 1 August 2019 (2019-08-01), pages 644-645, XP002796279, DOI: 10.1038/S41929-019-0323-6 * the whole document * ----- | 1-15 | TECHNICAL FIELDS SEARCHED (IPC) C12P C07D A61P A61K C12N |
| Y | JANIS FRICKE ET AL: "Enzymatic Synthesis of Psilocybin", ANGEWANDTE CHEMIE INTERNATIONAL EDITION, vol. 56, no. 40, 25 August 2017 (2017-08-25), pages 12352-12355, XP055583973, ISSN: 1433-7851, DOI: 10.1002/anie.201705489 * the whole document * ----- -/-- | 1-15 | |

The present search report has been drawn up for all claims

1

| Place of search | Date of completion of the search | Examiner |
|---|---|---|
| Berlin | 21 July 2022 | Schneider, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 15 4810

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KADOWAKI J. T. ET AL: "Copper oxide-based cathode for direct NADPH regeneration", SCIENTIFIC REPORTS, vol. 11, no. 1, 8 January 2021 (2021-01-08), XP055945147, DOI: 10.1038/s41598-020-79761-6 Retrieved from the Internet: URL:http://www.nature.com/articles/s41598-020-79761-6> * the whole document * ----- | 7,13 | |
| Y | HODGES M ET AL: "An iron regulatory-like protein expressed in Plasmodium falciparum displays aconitase activity", MOLECULAR AND BIOCHEMICAL PARASITOLOGY, ELSEVIER SCIENCE PUBLISHERS, AMSTERDAM, NL, vol. 143, no. 1, 1 September 2005 (2005-09-01), pages 29-38, XP027688664, ISSN: 0166-6851 [retrieved on 2005-09-01] * page 36 * ----- | 7,13 | TECHNICAL FIELDS SEARCHED (IPC) |
| Y,D | WO 2021/097452 A2 (VOGAN JACOB MICHAEL [US]; PEIFFER LAURA FLATAUER [US] ET AL.) 20 May 2021 (2021-05-20) * the whole document * ----- | 1-15 | |
| Y,D | WO 2021/086513 A1 (UNIV MIAMI [US]) 6 May 2021 (2021-05-06) * figure 1D * ----- | 1-15 | |

1

The present search report has been drawn up for all claims

| Place of search | Date of completion of the search | Examiner |
|---|---|---|
| Berlin | 21 July 2022 | Schneider, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 15 4810

This annex lists the patent family members relating to the patent documents cited in the above-mentioned European search report. The members are as contained in the European Patent Office EDP file on
The European Patent Office is in no way liable for these particulars which are merely given for the purpose of information.

21-07-2022

| Patent document cited in search report | Publication date | Patent family member(s) | Publication date |
|---|---|---|---|
| WO 2019180309 A1 | 26-09-2019 | CA 3106890 A1 | 26-09-2019 |
| | | EP 3768843 A1 | 27-01-2021 |
| | | FI 20185254 A1 | 20-09-2019 |
| | | US 2021010015 A1 | 14-01-2021 |
| | | WO 2019180309 A1 | 26-09-2019 |
| WO 2021097452 A2 | 20-05-2021 | CA 3155976 A1 | 20-05-2021 |
| | | US 2021147888 A1 | 20-05-2021 |
| | | WO 2021097452 A2 | 20-05-2021 |
| WO 2021086513 A1 | 06-05-2021 | AU 2020374768 A1 | 12-05-2022 |
| | | CA 3158505 A1 | 06-05-2021 |
| | | IL 292590 A | 01-06-2022 |
| | | WO 2021086513 A1 | 06-05-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019180309 A1
- WO 2019173797 A
- WO 2021086513 A1
- US 2021147888 A1


### Non-patent literature cited in the description


- **ADAY, JACOB S. ; MITZKOVITZ, CAYLA M. ; BLOESCH, EMILY K. ; DAVOLI, CHRISTOPHER C. ; DAVIS, ALAN K.** Long-Term Effects of Psychedelic Drugs: A Systematic Review. *Neuroscience and Biobehavioral Reviews,* June 2020, vol. 113, 179-89
- **NUTT, DAVID ; ERRITZOE, DAVID ; CARHART-HARRIS, ROBIN.** Psychedelic Psychiatry's Brave New World. *Cell,* 2020, https://doi.org/10.1016/j.cell.2020.03.020
- **FRICKE, JANIS ; BLEI, FELIX ; HOFFMEISTER, DIRK.** Enzymatic Synthesis of Psilocybin. *Angewandte Chemie,* 2017, vol. 56 (40), 12352-55
- **FRICKE, JANIS ; KARGBO, ROBERT ; REGESTEIN, LARS ; LENZ, CLAUDIUS ; PESCHEL, GUNDELA ; ROSENBAUM, MIRIAM A. ; SHERWOOD, ALEXANDER.** Scalable Hybrid Synthetic/Biocatalytic Route to Psilocybin. *Chem. Eur. J,* 2020, vol. 26, 8281-8285
- **DEMMLER, RICHARD ; FRICKE, JANIS ; DÖRNER, SEBASTIAN ; GRESSLER, MARKUS ; HOFFMEISTER, DIRK.** S-Adenosyl-L-Methionine Salvage Impacts Psilocybin Formation in 'Magic' Mushrooms. *Chembiochem: A European Journal of Chemical Biology,* 2020, vol. 21 (9), 1364-71
- **DEAN, JON G.** Indolethylamine-N-Methyltransferase Polymorphisms: Genetic and Biochemical Approaches for Study of Endogenous N,N,-Dimethyltryptamine. *Frontiers in Neuroscience,* 2018, https://doi.org/10.3389/fnins.2018.00232
- **NELSON, D. L. ; DAVID L. ; COX, M. M. ; HOSKINS, A. A. ; LEHNINGER, A. L.** *Lehninger Principles of Biochemistry,* vol. 3
- **PHARKYA, P. ; NIKOLAEV, E. V. ; MARANAS, C. D.** *Review of the BRENDA Database. Metabolic engineering,* 2003, vol. 5 (2), 71-73
- **SCHOMBURG, I. ; CHANG, A. ; SCHOMBURG, D.** BRENDA, Enzyme Data and Metabolic Information. *Nucleic Acids Research,* 2002, vol. 30 (1), 47
- **WITTIG, U. ; KANIA, R. ; GOLEBIEWSKI, M. ; REY, M. ; SHI, L. ; JONG, L. ; ALGAA, E. ; WEIDEMANN, A. ; SAUER DANZWITH, H. ; MIR, S.** SABIO-RK-Database for Biochemical Reaction Kinetics. *Nucleic Acids Research,* 2012, vol. 40, D790
- **HOOPS, S. ; GAUGES, R. ; LEE, C. ; PAHLE, J. ; SIMUS, N. ; SINGHAL, M. ; XU, L. ; MENDES, P. ; KUMMER, U.** COPASI-a COmplex PAthway Simulator. *Bioinformatics,* 2006, vol. 22 (24), 3067-3074
- **MENDES, P. ; HOOPS, S. ; SAHLE, S. ; GAUGES, R. ; DADA, J. ; KUMMER, U.** Computational Modeling of Biochemical Networks Using COPASI. *Methods in Molecular Biology,* 2009, vol. 500 (1), 17-59
- Simulation of biochemical networks using COPASI | Proceedings of the 38th conference on Winter simulation. *Proceedings of the 38th conference on Winter simulation,* 21 January 2022, https://dl.acm.org/doi/10.5555/1218112.1218421